# EUROPEAN PATENT SPECIFICATION

(11) **EP 3 199 948 B1**
(45) Date of publication and mention of the grant of the patent: **11.03.2020**
(21) Application number: 15843546.1
(22) Date of filing: 09.09.2015
(51) Int. Cl.: G01N 33/543, C12Q 1/68

(54) **METHOD FOR DETECTING TARGET MOLECULE, AND KIT FOR USE IN SAID METHOD**
VERFAHREN ZUR BESTIMMUNG EINES ZIELMOLEKÜLS UND KIT ZUR VERWENDUNG IN DIESEM VERFAHREN
PROCÉDÉ DE DÉTECTION DE MOLÉCULE CIBLE, ET TROUSSE DESTINÉE À ÊTRE UTILISÉE DANS LEDIT PROCÉDÉ

(30) Priority: 22.09.2014 JP 2014192311
(43) Date of publication of application: 02.08.2017
(73) Proprietor: Japan Science and Technology Agency, Kawaguchi-shi, Saitama 332-0012 (JP)
(72) Inventor: NOJI, Hiroyuki, Tokyo 113-8656 (JP)
(74) Representative: Plasseraud IP
(86) International application number: PCT/JP2015/004577
(87) International publication number: WO 2016/047068

(56) References cited:
- WO-A2-2005/059509
- WO-A2-2009/097425
- JP-A- 2007 525 661
- JP-A- 2009 000 673
- JP-A- 2009 506 312
- US-A1- 2006 110 739
- US-A1- 2009 176 259
- US-A1- 2014 228 239
- US-A1- 2014 228 239
- ROWLAND A M ET AL: "A simple approach to improving sensitivity in a one-step monoclonal antibody-based ELISA for human IIbIIIa using multiple conjugates", JOURNAL OF IMMUNOLOGICAL METHODS, ELSEVIER SCIENCE PUBLISHERS B.V.,AMSTERDAM, NL, vol. 151, no. 1-2, 6 July 1992 (1992-07-06), pages 87-95, XP023657576, ISSN: 0022-1759, DOI: 10.1016/0022-1759(92)90106-4 [retrieved on 1992-07-06]

## Description

### Technical Field

The present invention relates to a method for detecting a target molecule and a kit for use in the method. More specifically, the present invention relates to a method for detecting a target molecule based on an antigen-antibody reaction between a target molecule on a carrier and an antibody.

### Background Art

For diagnosing various diseases at early stages, a biosensing technology is desired for highly sensitively detecting disease markers (biomarkers) present at low concentrations in biological samples. Provided that 100 biomarker molecules are secreted in 5 L of blood from each of 1 million cancer cells contained in a tumor of 1 mm³ in volume, the concentration of the biomarker in blood is presumed to be about 30 aM. Development of a technology which enables detection of such a target molecule at a low concentration has been desired.

A method for detecting a protein by using single-molecule enzyme-linked immunosorbent assay (ELISA) is described in Non-Patent Literature 1. In this method, a trace amount of protein is captured by microbeads covered with an antibody specific to the protein; and complexs formed of the beads and the protein are labeled with fluorescence. Beads containing the complexs are introduced in a reaction chamber with the help of centrifugal force and the number of beads capturing the protein is counted. In this manner, the protein is quantitatively measured.

Non-Patent Literature 2 discloses an approach to improving sensitivity in a one-step monoclonal antibody-based ELISA for human IIbIIIa using multiple conjugates.

Patent Literature 1 discloses a "single-molecule digital counting device", which is an array device enabling formation of microdroplets with an extremely high density. Owing to ELISA carried out in a small-volume droplet, a signal from a target molecule is expressed by a binary value and subjected to the measurement (digital ELISA). To describe the method more specifically, first, a target molecule, beads modified with a capture antibody and a detection antibody are reacted to form a "capture antibody-target molecule-detection antibody" complex on the surface of the beads. When the concentration of the target molecule is low, each bead falls in one of two categories: binding only one complex or binding no complex. Then, the beads are enclosed one by one in a large number of microdroplets formed in the array device. The number of microdroplets emitting a signal derived from the detection antibody is counted and determined as the number of target molecules. In this manner, the signals from the target molecules are expressed by a binary value, either 0 or 1, with the result that detection and quantification of the target molecule can be made with a high sensitivity and a high accuracy.

In connection with the present invention, Patent Literature 2 discloses a method for detecting a target substance based on enzyme immunoassay. In this immunoassay, a restriction enzyme is used as a label to be attached to an antibody reacting with the target substance; a DNA chain having the nucleotide sequence which is to be cleaved by the restriction enzyme is cleaved by the restriction enzyme in a complex; and the cleaved DNA-chain fragments are analyzed (measured) to detect the target substance.

Patent literature 3 discloses molecular biosensors having one or more aptamers, which are useful in several methods including in the identification and quantification of target molecules.

Patent literature 4 discloses methods of detecting a target molecule in a sample comprising incubating the sample with two or more detectably labeled probes, partitioning the sample into multiple partitions, and detecting the presence of the two or more probes in the same partition.

Patent literature 5 discloses methods for detecting truncated molecules.

Patent literature 6 discloses compositions and methods that are useful in the identification and quantification of any polypeptide or macromolecular complex using a set of co-aptamer constructs.

### Citation List

### Patent Literature

Patent Literature 1: International Publication No. WO2012/121310
Patent Literature 2: Japanese Patent Laid-Open No. H7-270418
Patent literature 3: US 2006/110739
Patent literature 4: US 2014/228239
Patent literature 5: WO 2009/097425
Patent literature 6: WO 2005/059509

### Non-Patent Literature

Non-Patent Literature 1: David M Rissin et al., Nature Biotechnology: doi: 10.1038/nbt.1641
Non-Patent Literature 2 : Rowland, A. M. et al. Journal of Immunological Methods, 1992: doi: 10.1016/0022-1759(92)90106-4

### Summary of Invention

### Technical Problem

In the case where a target molecule is measured based on the single-molecule digital counting by ELISA as described above, when noise is derived from the detection antibody nonspecifically adsorbed to beads, a signal from a target molecule cannot be accurately binarized, with the result that quantitative performance decreases.

Then, a primary object of the present invention is to provide a technique for detecting a signal from a target molecule with a high sensitivity and a high accuracy, while eliminating noise derived from a detection antibody non-specifically adsorbed.

### Solution to Problem

The scope of the invention is defined by the claims. Any subject-matter falling outside the scope of the claims is provided for information purposes only

To attain the above object, the present description provides the following [1] to [8].
[1] A method for detecting a target molecule, including
   a complex formation step of reacting
   a target molecule,
   a carrier modified with a first antibody which specifically binds to the target molecule, and
   two or more second antibodies which specifically bind to the target molecule, are labeled with enzymes having a substrate cleaving activity and mutually different substrate specificities
   to form a complex consisting of the first antibody, the target molecule and the second antibodies on the carrier; and
   a detection step of reacting
   two or more substrates having cleavage sites by the enzymes, a fluorescent substance bound to one terminal side of each of the cleavage site and a quencher bound to another terminal side thereof, wherein the fluorescent substances are mutually different in fluorescence wavelength, and
   the complex
   to detect fluorescence emitted from the fluorescent substances.
[2] The detection method according to [1], including an analysis step of processing two or more detection signals of fluorescence different in wavelength as a detection signal of the target molecule.
[3] The detection method according to [1] or [2], including, between the complex formation step and the detection step, an enclosing step of enclosing the carriers one by one in droplets formed on the base plate.
[4] The detection method according to any one of [1] to [3], in which the carrier is a microbead.
[5] The detection method according to any one of [1] to [4], in which the first antibody and the second antibody bind to different epitopes of the target molecule.
[6] The detection method according to any one of [1] to [5], being digital ELISA.
[7] A method for detecting a target molecule, including
   a complex formation step of reacting
   a target molecule,
   a carrier modified with a first antibody which specifically binds to the target molecule, and
   two or more second antibodies which specifically bind to the target molecule and are provided with mutually different labels
   to form a complex consisting of the first antibody, the target molecule and the second antibodies on the carrier;
   a detection step of detecting the signals from the labels; and
   an analysis step of processing two or more different signals from the labels as a detection signal of the target molecule.
[8] An enzyme linked immunosorbent assay (ELISA) kit, containing
   a carrier modified with a first antibody which specifically binds to a target molecule,
   two or more second antibodies which specifically bind to the target molecule and are labeled with enzymes having a substrate cleaving activity and mutually different substrate specificities, and
   two or more substrates having cleavage sites by the enzymes, a fluorescent substance bound to one terminal side of each of the cleavage sites and a quencher bound to another terminal side thereof, wherein the fluorescent substances are mutually different in fluorescence wavelength.

### Advantageous Effects of Invention

According to the present invention, there is provided a method for detecting a target molecule based on an antigen-antibody reaction between the target molecule on a carrier and an antibody. In the method, a technique for detecting a signal from the target molecule with a high sensitivity and a high accuracy while eliminating noise derived from a detection antibody nonspecifically adsorbed to the carrier is provided.

### Brief Description of Drawings

[Figure 1] Figure 1 illustrates a complex formed in a complex formation step.
[Figure 2] Figure 2 illustrates microbeads enclosed in microdroplets in an enclosing step.
[Figure 3] Figure 3 illustrates the reaction between a complex and a probe in a detection step.
[Figure 4] Figure 4 illustrates a complex formed in a complex formation step.

### Description of Embodiments

Now, a preferred embodiment will be described below with reference to the accompanying drawings.

The method for detecting a target molecule according to the present description includes the following steps. Herein, the case where the method for detecting a target molecule according to the present description is applied to the single-molecule digital counting by ELISA (digital ELISA) will be described as an embodiment. Now, the individual steps thereof will be described.
(1) a complex formation step of reacting a target molecule, a carrier modified with a first antibody which specifically binds to the target molecule, and two or more second antibodies which specifically bind to the target molecule, are labeled with enzymes having a substrate cleaving activity and mutually different substrate specificities to form a complex consisting of the first antibody, the target molecule and the second antibodies on the carrier.
(2) an enclosing step of enclosing the carriers one by one in droplets formed on a base plate.
(3) a detection step of reacting two or more substrates having cleavage sites by the enzymes, a fluorescent substance bound to one terminal side of each of the cleavage sites and a quencher bound to another terminal side thereof, wherein the fluorescent substances are mutually different in fluorescence wavelength.
(4) an analysis step of processing two or more detection signals of fluorescence different in wavelength as the detection signal of the target molecule.

In the detection method according to the present description, a detection object, i.e., a target molecule, may be any substance as long as the substance binds to an antibody through an antigen-antibody reaction. Particularly, the target molecule is specified as a microorganism such as a bacterium and a fungus and a biomolecule such as a virus, a protein, a nucleic acid, a sugar and a complex of these. The target molecule (a detection object) is not limited to one. Two or more target molecules can be simultaneously detected. For example, if four antibodies: first and second antibodies against protein A and first and second antibodies against protein B are used, two target molecules, i.e., protein A and protein B can be distinguishably and simultaneously detected.

### 1. Complex formation step

In the complex formation step, a target molecule, a carrier modified with a first antibody which specifically binds to the target molecule, and a second antibody which specifically binds to the target molecule and is labeled with an enzyme having a substrate cleaving activity are reacted to form a complex consisting of the first antibody, the target molecule and the second antibody on the carrier. As the second antibody, two or more antibodies labeled with enzymes different in substrate specificity are used.

The "enzyme having a substrate cleaving activity" is not particularly limited as long as it can cleave a substrate and realize dissociation of a fluorescent substance and a quencher (specifically described later). As the "enzyme having a substrate cleaving activity", for example, transferases classified in EC2 (EC stands for Enzyme Commission Number) and hydrolases classified in EC3 and lyases classified in EC4 can be used. Specific names of enzymes and their substrates (and cleavage sites in the substrates) to be used in combination are as follows.

**[Table 1]**

| | |
|---|---|
| Enzyme | Substrate (cleavage site in substrate) |
| Esterase | Ester bond or bond derived from ester bond |
| Glucosidase | Glycoside bond or bond derived from glycoside bond |
| Phosphatase | Phosphate bond or bond derived from phosphate bond |
| DNAse | DNA and derivative thereof |
| RNAse | RNA and derivative thereof |
| Protease | Peptide bond and bond derived from peptide bond |

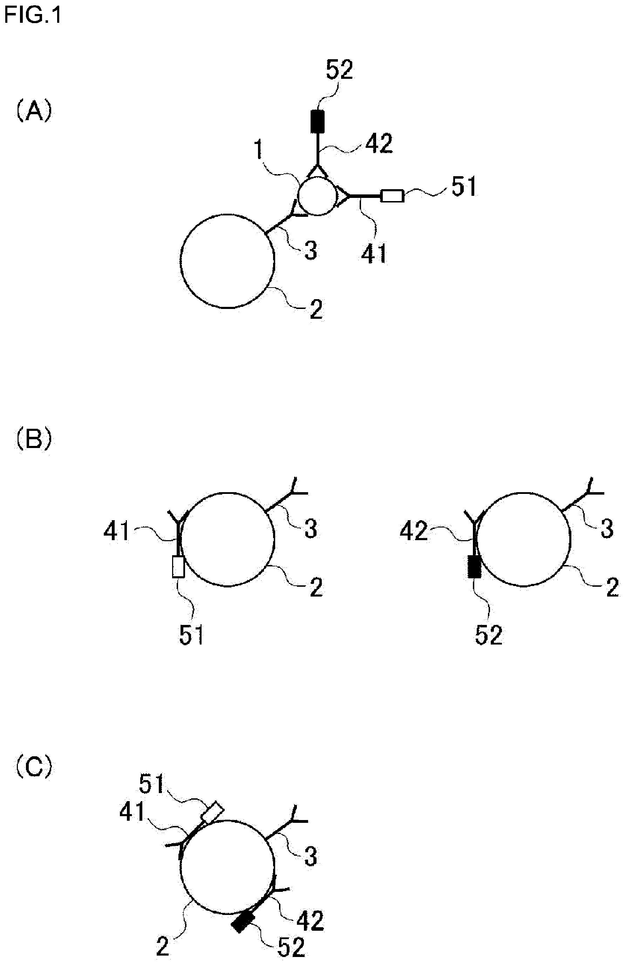

Figure 1 shows a complex formed in the complex formation step. In this step, first, a carrier 2 modified with a first antibody 3 specifically binding to a target molecule 1 and second antibodies 41, 42 specifically binding to the target molecule 1 and labeled with enzymes 51, 52 are prepared.

The "antibody specifically binding" means that antibody can bind to an antigen (herein, target molecule 1) and does not bind or weakly binds to other substances. The "weakly binds" means that binding affinity for other substances is distinguishably low compared to the binding affinity for the antigen. The binding affinity for the antigen can be measured by a method known in the art, for example, surface plasmon resonance (SPR).

The first antibody 3 functions to capture the target molecule 1 on the carrier 2. The second antibodies 41, 42 function to allow optical detection of the target molecule 1 captured on the carrier 2. It is preferable that the first antibody 3 and the second antibodies 41, 42 bind different epitopes of the target molecule 1. In other words, it is preferable that the epitopes recognized by the first antibody 3, second antibody 41 and second antibody 42 are all different. Hereinafter, the first antibody 3 will be referred also to as the "capture antibody 3" and the second antibodies as the "detection antibodies 41, 42".

As the carrier 2, microbeads are widely used. Hereinafter, the "carrier 2" will be referred to also as the "microbead 2". The "microbead", which is used synonymously with a "particle", is a technical term commonly used in the art. The shape of the microbead is not particularly limited but usually spherical. The material for the microbead is not particularly limited and may be, e.g., glass, silica gel, polystyrene, polypropylene, membrane and a magnetic material. Examples of specific materials include cellulose, cellulose derivatives, acrylic resins, glass, silica gel, polystyrene, gelatin, polyvinylpyrrolidone, vinyl-acrylamide copolymers, polystyrenes crosslinked with e.g., divinylbenzene, polyacrylamide, latex gel, polystyrene dextran, rubber, silicon, plastic, nitrocellulose, cellulose, natural sponge, silica gel, glass, metal plastic, cellulose, crosslinked dextran (Sephadex (trademark)) and agarose gel (Sepharose (trademark)). The bead may be porous. The beads preferably have an average particle diameter of 5 µm or less, for example, about 1 µm to 4 µm. Note that, the average particle diameter can be measured, for example, by electron microscopic observation or a dynamic light scattering method.

A microbead 2 is modified with a capture antibody 3 by binding the capture antibody 3 to a group (modifying group) present on the surface of the microbead 2 via a linker. For example, the capture antibody 3 is covalently bound to an amino group present on the surface of an amino group-modified bead, via a crosslinking agent having, e.g., N-hydroxysuccinimide.

The enzymes 51, 52 to be labeled on the detection antibodies 41, 42 are defined as enzymes different in substrate specificity. The "substrate specificity" herein means that, in cleaving the substrate catalyzed by an enzyme, the enzyme does not catalyze cleavage of substances other than the substrate or its catalytic action is fully weak. The "enzymes different in substrate specificity" means that if esterase is used as the enzyme 51, an enzyme such as glucosidase and phosphatase, which does not cleave an ester bond, is used as the enzyme 52.

In the case where a combination of a restriction enzyme and a nucleic acid chain is employed as a combination of an enzyme and a substrate, enzymes different in recognition sequence (cleavage site) are used as the enzymes 51, 52 different in substrate specificity. Examples of the restriction enzyme include AccI, AluI, ApaI, BamHI, BglII, BssHII, BstEII, ClaI, DdeI, DraI, EcoRI, EcoRV, HaeIII, HincII, HindIII, HpaI, HpaII, KpnI, MluI, NarI, NcoI, NdeI, NheI, NotI, PstI, PvuI, PvuII, RsaI, SacI, Sail, ScaI, SmaI, SpeI, SphI, SspI, StuI, XbaI and XhoI. As the enzymes 51, 52, two enzymes different in substrate specificity can be arbitrarily selected from these and used in combination. Hereinafter, a case where restriction enzymes are used as the enzymes 51, 52 will be mainly described. The Enzymes 51, 52 will be also referred to as the "restriction enzymes 51, 52".

The detection antibodies 41, 42 can be labeled with the restriction enzymes 51, 52, respectively, by forming a crosslinked structure between the detection antibodies 41, 42 and the restriction enzymes 51, 52, respectively, by use of a crosslinking agent (crosslinker reagent).

Next, in this step, the target molecule 1, the microbead 2 modified with the capture antibody 3, and the detection antibodies 41, 42 labeled with the restriction enzymes 51, 52 are reacted. As a result of the reaction, a complex consisting of the capture antibody 3, the target molecule 1 and the detection antibodies 41, 42 is formed on the microbead 2 (see, Figure 1A). The target molecule 1, microbead 2 and detection antibodies 41, 42 may be reacted in a single step or two steps. In other words, the target molecule 1, microbead 2 and detection antibodies 41, 42 may be simultaneously reacted. Alternatively, the target molecule 1 is reacted with the microbead 2, and then the microbead 2 is washed in order to remove the target molecule 1 which did not bind to the capture antibody 3, and thereafter, the microbead 2 may be reacted with the detection antibodies 41, 42.

The target molecule 1, microbead 2 and detection antibodies 41, 42 may be reacted in an appropriate solution by bringing them into contact with one another in the same conditions as in a conventional enzyme linked immunosorbent assay. When the concentration of the target molecule 1 is low, each microbead 2 after the reaction falls in one of the categories: having a single complex alone and having no complex.

In the detection step (described later), a microbead 2 having the complex (formed in this step) on the surface is allowed to be in contact with substrates each having a fluorescent substance bound thereto. The substrates are cleaved, respectively, by the restriction enzymes 51, 52 provided as labels to the detection antibodies 41, 42. As a result, fluorescence is emitted and detected. At this time, if the detection antibodies 41, 42 nonspecifically adsorb to the microbeads 2, fluorescence is emitted also from the microbeads 2 having detection antibodies 41, 42 nonspecifically adsorbed onto the surface by substrate cleavage.

Herein, the "antibody nonspecifically adsorbs" means that an antibody adsorbs to a non-antigen part of a substance containing an antigen and to a substance containing no antigen, in short, refers to absorption to a substance not via an antigen-antibody reaction.

Figures 1B and C show nonspecific adsorption of the detection antibodies 41, 42 to the microbeads 2 occurring in the complex formation step. Figure 1B shows the states where the detection antibody 41 and detection antibody 42 nonspecifically adsorb separately to the surfaces of microbeads 2. Figure 1C shows the case where both detection antibody 41 and detection antibody 42 nonspecifically adsorb to the surface of a microbead 2. In the complex formation step, nonspecific adsorption of detection antibodies 41, 42 as shown in Figures 1B and C may occur, in addition to the desired formation of a complex as shown in Figure 1A

The nonspecific adsorption by both detection antibody 41 and detection antibody 42 as shown in Figure 1C occurs at a sufficiently low frequency, compared to the nonspecific adsorption of either one of them as shown Figure 1B and substantially produces no effect on detection accuracy of the target molecule 1. For example, assuming that nonspecific adsorption of the detection antibody 41 occurs in 1% of the microbeads 2 and nonspecific adsorption of the detection antibody 42 occurs 1% of the microbeads 2, the frequency of occurrence of nonspecific adsorption of both detection antibody 41 and detection antibody 42 is only 0.01%. In the analysis step described later, two or more fluorescence detection signals different in (fluorescence) wavelength are processed as a detection signal of the target molecule 1. In this manner, noise derived from a detection signal of fluorescence due to nonspecific adsorption as shown in Figure 1B is eliminated.

### 2. Enclosing step

In the enclosing step, microbeads 2 are enclosed in droplets formed on a base plate. This step is carried out when the method for detecting a target molecule according to the present description is applied to digital ELISA and is not an essential step for the detection method according to the present description.

In this step, microbeads 2 are enclosed one by one in droplets each having a volume small enough to contain only one microbead 2, in order to express a signal from the target molecule 1 by a binary value, i.e., 0 or 1, in the analysis step. In formation of the microdroplets and enclosure of microbeads 2 in the microdroplets one by one, a single molecule digital counting device disclosed, for example, in Patent Literature 1, can be suitably used. According to the single molecule digital counting device, microdroplets are extremely densely formed on a base plate; at the same time, microbeads 2 can be enclosed in the droplets. After the complex formation step, microbeads 2 are washed to remove detection antibodies 41, 42 which failed to bind to the target molecule 1, and then resuspended in an appropriate solvent and may be subjected to this step.

After the complex formation step, a mixture of microbeads forming complexs (see, Figure 1A) and microbeads not forming complexs is obtained. In the microbeads not forming complexs, microbeads to which detection antibodies 41, 42 are nonspecifically adsorbed are included (see, Figure 1B, C).

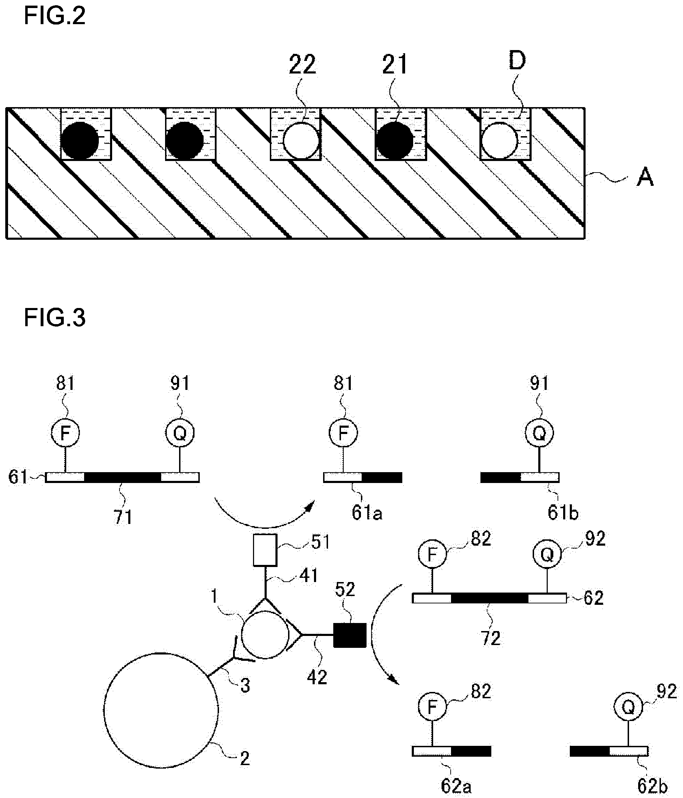

Figure 2 shows microbeads 2 enclosed in microdroplets. Microbeads 2 are enclosed one by one in droplets D formed on base plate A. At the reaction in the complex formation step, if the concentration of the target molecule 1 is low, each microbead 2 falls in one of the categories: having a single complex and having no complex. In the figure, the microbeads on the surface of which a complex is formed are indicated by reference number 21; whereas the microbeads having no complex formed thereon are indicated by reference number 22. The state of the microbead 21 is shown in Figure 1A and the state of the microbead 22 is shown in Figure 1B or C. Hereinafter, the microbead 2 having a complex formed thereon will be referred to as "microbead 21"; whereas, the microbead 2 having no complex formed thereon will be referred to as "microbead 22".

### 3. Detection step

In the detection step, substrates (hereinafter also referred to as "probes"), each of which has a recognition sequence by the restriction enzyme 51, 52, a fluorescent substance bound to one terminal side of the recognition sequence (i.e., a cleavage site) and a quencher bound to another terminal side thereof, are reacted with the complexs formed on the surfaces of microbeads 2 in the complex formation step and then fluorescence emitted from fluorescent substances is detected.

Figure 3 shows the reaction between a probe and a complex in this step. In the reaction, two or more probes are used, to which fluorescent substances having different fluorescence wavelengths are separately bound. More specifically, a probe indicated by reference numeral 61 in the figure has a cleavage site 71 by a restriction enzyme 51, which is provided as a label to a detection antibody 41. To one of the regions sandwiching the cleavage site 71, a fluorescent substance 81 is bound; and a quencher 91 is bound to the other region. The probe indicated by reference numeral 62 in the figure has a cleavage site 72 by a restriction enzyme 52, which provided as a label to a detection antibody 42. To one of the regions sandwiching the cleavage site 72, a fluorescent substance 82 is bound; and a quencher 92 is bound to the other region.

Since the restriction enzymes 51, 52 are different in substrate specificity, the nucleotide sequences of the cleavage sites 71, 72 are mutually different. As the fluorescent substances 81, 82 for probes 71, 72, fluorescent substances different in fluorescence wavelength optically distinguishably detected are used.

Herein, when a combination other than a combination of a restriction enzyme and a nucleic acid chain is employed as the combination of an enzyme and a substrate, for example, esterase is used as an enzyme 51 and glucosidase is used as an enzyme 52, a probe containing a cleavage site 71 (ester bond) by esterase provided as a label to a detection antibody 41, a fluorescent substance 81 bound to one of the regions sandwiching the cleavage site 71 and a quencher 91 bound to the other region is used as the probe 61; and a probe containing a cleavage site 72 (glycoside bond) by glucosidase provided as a label to a detection antibody 42, a fluorescent substance 82 bound to one of the regions sandwiching the cleavage site 72 and a quencher 92 bound to the other region is used as the probe 62.

The quenchers 91, 92 are present within a certain distance from the fluorescent substances 81, 82 such that energy can be transferred between them, and prevent (quench) light emission from the fluorescent substances 81, 82. As the fluorescent substances 81, 82 and the quenchers 91, 92, a fluorescent substance and a quencher commonly used in optical detection technology for nucleic acids, such as real-time quantitative PCR, can be used. As a combination of a fluorescent substance and a quencher, for example, a combination of a fluorescent substance selected from the group consisting of Alexa Fluor (registered trade mark) 488 (manufactured by Invitrogen), ATTO 488 (manufactured by ATTO-TEC GmbH), Alexa Fluor (registered trade mark) 594 (manufactured by Invitrogen) and ROX (Carboxy-X-rhodamine), and a BHQ (registered trade mark, Black hole quencher)-1 or BHQ (registered trade mark)-2, may be mentioned. In addition, e.g., a combination of fluorescein and DABCYL can be mentioned. Combinations of a fluorescent substance and a quencher commonly used are shown in the following table.

**[Table 2]**

| Fluorescent substance | Maximum excitation wavelength (nm) | Maximum fluorescence wavelength (nm) | Quencher |
|---|---|---|---|
| 6-FAM™ | 494 | 515 | BHQ-1, DABCYL |
| Fluorescein | 495 | 520 | BHQ-1, DABCYL |
| JOE™ | 520 | 548 | BHQ-1, DABCYL |
| TET™ | 521 | 536 | BHQ-1, DABCYL |
| HEX™ | 353 | 555 | BHQ-1, DABCYL |
| Cyanine 3 | 550 | 570 | BHQ-2, DABCYL |
| ROX™ | 573 | 602 | BHQ-2, DABCYL |
| Texas RED (registered trademark) | 583 | 603 | BHQ-2, DABCYL |
| Cyanine 5 | 651 | 674 | BHQ-3, DABCYL |
| Cyanine 5.5 | 675 | 694 | BHQ-3, DABCYL |

The reaction is carried out by bringing probes 61, 62 into contact with microbeads 2 enclosed in microdroplets D. More specifically, after the complex formation step, microbeads 2 are washed and resuspended in a solution containing probes 61, 62. In this manner, these are allowed to be contact with each other. The reaction is preferably carried out a buffer having an appropriate composition in accordance with the types of restriction enzymes 51, 52. Microdroplets are preferably formed of such a buffer previously in the enclosing step. Note that buffers optimized for restriction enzymes are set in combination with restriction enzymes and commercially available.

In the case where a microbead 21 is enclosed in a microdroplet D, the cleavage site 71 of a probe 61 is cleaved by a restriction enzyme 51 provided as a label to a detection antibody 41 forming a complex. When the cleavage site 71 is cleaved, the probe 61 is cut into a fragment 61a and a fragment 61b, with the result that a fluorescent substance 81 dissociates from a quencher 91 and falls in the state where light can be emitted. Similarly, when the cleavage site 72 of a probe 62 is cleaved by a restriction enzyme 52 provided as a label to a detection antibody 42 forming a complex, a fluorescent substance 82 dissociates from a quencher 92 and falls in the state where light can be emitted.

Fluorescence emitted from individual microdroplets enclosing microbeads 2 is detected by using, e.g., a fluorescence microscope and an image sensor.

In this step, it is preferable to detect whether or not a microbead 2 is contained in a microdroplet. The presence or absence of a microbead 2 can be checked by observation of a microbead, for example, by a microscope and also by employing e.g., a method for detecting light scattered by a microbead 2, or a potential measurement method by a field effect transistor (FET).

### 4. Analysis step

In the analysis step, two or more detection signals of fluorescence having different fluorescence wavelengths are processed as the detection signal of a target molecule 1. The number of microdroplets D emitting the detection signal of a target molecule 1 is counted and specified as the number of target molecules.

At the time of a reaction in the complex formation step, when the concentration of the target molecule 1 is low, each of the microbeads 2 enclosed in microdroplets D is either microbead 21 having a single complex alone or a microbead 22 having no complex. Thus, the number of microdroplets D sending detection signal of the target molecule 1 can be regarded as the number of the target molecules 1. Based on the number of microdroplets D enclosing microbeads 21 and microbeads 22 and the number of microdroplet D enclosing microbeads 21, the ratio of microbeads 2 which capture the target molecule 1 relative to the total number of microbeads 2 can be calculated. In this way, the concentration of the target molecule can be quantified.

As described above, if a microbead 21 is enclosed in a microdroplet D, fluorescence from a fluorescent substance 81 and fluorescence from a fluorescent substance 92 mutually different in wavelength are detected. As shown in Figure 1B, in the case of a microbead 22, which has a detection antibody 41 or a detection antibody 42 just nonspecifically adsorbed to the surface and forms no complex, fluorescence is only detected from either one of the fluorescent substance 81 and fluorescent substance 92. Accordingly, if fluorescence detection signals from both of the fluorescent substance 81 and fluorescent substance 92 are processed as the detection signal from the target molecule 1, noise derived from the fluorescence detection signal derived from a microbead 22 forming no complex can be significantly reduced. In this manner, binarization of the detection signal of the target molecule 1 can be highly accurately carried out and the quantification of the target molecule 1 can be improved.

Note that, in the case where both a detection antibody 41 and a detection antibody 42 are nonspecifically adsorbed onto the surface of a microbead 2, as shown in Figure 1C, fluorescence is emitted from both fluorescent substance 81 and fluorescent substance 92; however, as already described, since the frequency of occurrence of nonspecific adsorption by both detection antibody 41 and detection antibody 42 is sufficiently low, the fluorescence has little influence on quantification of the target molecule 1.

Microbeads 2 and detection antibodies 41, 42 (see, Figure 1) and probes 71, 72 (see, Figure 3) used in the embodiment are set in a kit and preferably used for carrying out the method for detecting a target molecule according to the present description. More specifically, according to an aspect of the present description, there is also provided an enzyme linked immunosorbent assay (ELISA) kit, containing
(i) a carrier modified with a first antibody which specifically binds to a target molecule,
(ii) two or more second antibodies which specifically bind to the target molecule and are labeled with enzymes having a substrate cleaving activity and mutually different substrate specificities, and
(iii) two or more substrates having cleavage sites by the enzymes, a fluorescent substance bound to one terminal side of each of the cleavage sites and a quencher bound to another terminal side thereof, wherein the fluorescent substances are mutually different in fluorescence wavelength.

In the kit, for the microbead 2, a microbead modified with a capture antibody 3 (first antibody) in advance may be employed or an antibody may be attached to a modifying group present on the surface of the beads via a linker when used. Detection antibodies 41, 42 (second antibody) may be provided with enzymes as label in advance or an enzyme may be attached to the antibody by use of a crosslinking agent when used.

The kit according to the present description further contains e.g., reagents such as a crosslinking agent for use in modification with microbeads 2 with a capture antibody 3 or attaching an enzyme as a label to detection antibody 41, 42, buffers used in the complex formation step and the detection step and a base plate A (see, Figure 2) used in the enclosing step.

In the embodiment described above, two detection antibodies and two probes corresponding to these are used; and noise derived from nonspecific adsorption of a detection antibody(s) is reduced by processing two detection signals of fluorescence different in wavelength as a detection signal of a target molecule. In the method for detecting a target molecule according to the present description, three or more pairs of detection antibodies and probes may be used. In this case, three or more detection signals of fluorescence different in wavelength may be processed as a detection signal of a target molecule. As the number of detection antibodies and probes increases, an effect of reducing noise derived from nonspecific adsorption of a detection antibody(s) can be enhanced.

In the embodiment described above, a detection antibody labeled with an enzyme having a substrate cleaving activity is used as the second antibody; and fluorescence is emitted by cutting a cleavage site of a probe with the enzyme. In the method for detecting a target molecule according to the present description, a detection antibody labeled with an enzyme conventionally used in chemical color development and a detection antibody labeled with a fluorescent dye, can be used as the second antibody.

More specifically, the present description also encompasses a method for detecting a target molecule including the following steps, as a second embodiment.
(A) a complex formation step of reacting
   a target molecule, a carrier modified with a first antibody which specifically binds to the target molecule, and two or more second antibodies which specifically bind to the target molecule and are provided with mutually different labels
   to form a complex consisting of the first antibody, the target molecule and the second antibodies on the carrier.
(B) a detection step of detecting the signals from the labels.
(C) an analysis step of processing the signals from two or more different labels as a detection signal from the target molecule.

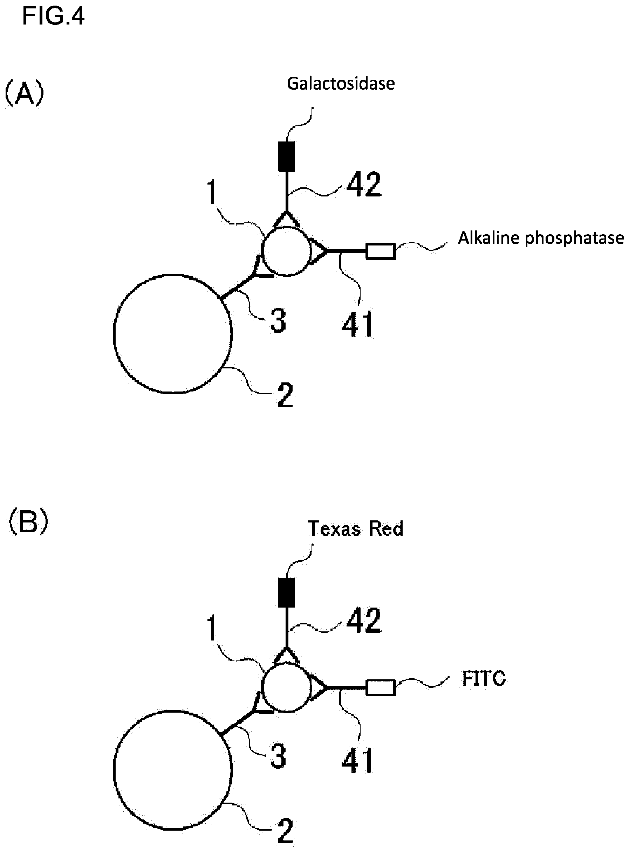

In the step (C) herein, the "signals from labels" include signals directly and indirectly emitted from the labels. More specifically, in the case where detection antibodies labeled with fluorescent dyes are used as the second antibodies, the "signals from labels" refer to fluorescence emitted from the fluorescent dyes (see, Figure 4B). Also in the case where detection antibodies labeled with enzymes for use in chemical color development are used as the second antibodies, the "signals from labels" refer to chemical color development by the catalytic action of the enzymes (see, Figure 4A).

The step (A) can be carried out in the same manner as in step (1) of the aforementioned embodiment (first embodiment) except that detection antibodies which are labeled with enzymes, such as alkaline phosphatase and galactosidase, conventionally used in chemical color development or with fluorescent substances, are used as the second antibodies. If the embodiment is applied to digital ELISA, the enclosing step described as the step (2) in the first embodiment may be included. A detection antibody can be labeled with an enzyme or a fluorescent dye in accordance with the aforementioned method known in the art. Alternatively, a commercially available antibodies labeled with enzymes or fluorescent dyes may be used.

In the step (B), signals from the labels attached to detection antibodies forming a complex on the surface of a carrier are detected. Figure 4A shows a complex consisting of "capture antibody 3-target substance 1-second antibodies 41, 42" formed on microbead 2 in the case where an antibody labeled with alkaline phosphatase and an antibody labeled with galactosidase are used as detection antibodies 41, 42. When a detection antibody labeled with an enzyme is used, a signal can be detected by developing a color using a substrate of the enzyme. For example, in the case of a detection antibody labeled with alkaline phosphatase, detection can be made by reacting a complex with BCIP (5-Bromo-4-chloro-3-indolyl-phosphate) or NBT (4-mitro blue tetrazolium chloride), which is a chromogenic substrate for alkaline phosphatase in place of the probe used in the first embodiment. In the case of a detection antibody labeled with galactosidase, a chromogenic substrate such as X-Gal (5-bromo-4-chloro-3-indolyl-β-D-galactopyranoside) is used. As the detection antibody, two or more antibodies labeled with mutually different enzymes are used. As the chromogenic substrate, two or more compounds are used depending upon the enzymes provided as a label to individual antibodies. Signals derived from individual enzymes can be detected by measuring the color emitted from each of the chromogenic substrates by an absorption spectrometer.

When a detection antibody labeled with a fluorescent substance is used, fluorescence emitted from a fluorescent substance is detected by use of a fluorescence microscope or an image sensor. Figure 4B shows a complex consisting of "capture antibody 3-target substance 1-second antibodies 41, 42" formed on a microbead 2 in the case where an antibody labeled with FITC and an antibody labeled with Texas Red (registered trademark) are used as detection antibodies 41, 42. As the detection antibody, two or more antibodies labeled with fluorescent substances having mutually different fluorescence wavelengths are used and signals derived from individual fluorescent substances can be detected by specifying the wavelength zones of the fluorescence from the fluorescent substances.

In the step (C), signals from two or more different labels (alkaline phosphatase and galactosidase or FITC and Texas Red in the aforementioned cases) are processed as the detection signal of a target molecule. As described above, in the case where detection antibodies are only nonspecifically adsorbed onto the surface of a carrier and do not form a complex, any one of signals from two or more labels is only detected (see, Figure 1). Accordingly, if signals from two or more different labels are processed as the detection signal of a target molecule, noise derived from a detection antibody(s) nonspecifically adsorbed to a carrier can be significantly reduced. In this manner, detection accuracy of a target molecule as well as quantification can be improved.

### Reference Signs List

1: Target molecule, 2: Microbeads (carrier), 3: Capture antibody (first antibody), 41, 42: detection antibody (second antibody), 51, 52: Restriction enzyme, 61,62: Probe, 71,72: Cleavage site, 81,82: Fluorescent substance, 91,92: Quencher

## Claims

1. A method to detect a target molecule, comprising a complex formation step of reacting
a target molecule,
a carrier modified with a first antibody which specifically binds to the target molecule, and
two or more second antibodies which specifically bind to the target molecule and are labeled with enzymes having a substrate cleaving activity and mutually different substrate specificities
to form a complex consisting of the first antibody, the target molecule and the second antibodies on the carrier, wherein the first antibody and the second antibody bind to different epitopes of the target molecule;
a detection step of reacting
two or more substrates having cleavage sites by the enzymes, a fluorescent substance bound to one terminal side of each of the cleavage sites and a quencher bound to another terminal side thereof, wherein the fluorescent substances are mutually different in fluorescence wavelength, and
the complex
to detect fluorescence emitted from the fluorescent substances; and
an analysis step of processing two or more detection signals of fluorescence different in wavelength as a detection signal of the target molecule.

2. The detection method according to Claim 1, wherein the carrier is a microbead.

3. The detection method according to Claims 1 or 2, being digital ELISA.

4. The detection method according to Claim 3, comprising, between the complex formation step and the detection step, an enclosing step of enclosing the carriers one by one in droplets formed on a base plate.

5. A method to detect a target molecule, comprising a complex formation step of reacting
a target molecule,
a particle modified with a first antibody which specifically binds to the target molecule, and
two or more second antibodies which specifically bind to the target molecule and are provided with mutually different labels
to form a complex consisting of the first antibody, the target molecule and the second antibodies on the particle, wherein the first antibody and the second antibody bind to different epitopes of the target molecule;
a detection step of detecting the signals from the labels; and an analysis step of processing two or more different signals from the labels as a detection signal of the target molecule.

6. The detection method according to Claim 5, being digital ELISA.

7. The detection method according to Claim 6, comprising, between the complex formation step and the detection step, an enclosing step of enclosing the particles one by one in droplets formed on a base plate.

8. An enzyme linked immunosorbent assay (ELISA) kit, comprising
a carrier modified with a first antibody which specifically binds to a target molecule,
two or more second antibodies which specifically bind to the target molecule and are labeled with enzymes having a substrate cleaving activity and mutually different substrate specificities, wherein the first antibody and the second antibody bind to different epitopes of the target molecule, and
two or more substrates having cleavage sites by the enzymes, a fluorescent substance bound to one terminal side of each of the cleavage sites and a quencher bound to another terminal side thereof, wherein the fluorescent substances are mutually different in fluorescence wavelength.

## Patentansprüche

1. Verfahren zum Nachweis eines Zielmoleküls, umfassend einen Komplexbildungsschritt Umsetzen von
einem Zielmolekül,
einem Träger, der mit einem ersten Antikörper modifiziert ist, der spezifisch an das Zielmolekül bindet, und
zwei oder mehr zweiten Antikörpern, die spezifisch an das Zielmolekül binden und mit Enzymen markiert sind, die eine Substratspaltungsaktivität und gegenseitig unterschiedliche Substratspezifitäten aufweisen,
um einen Komplex zu bilden, der aus dem ersten Antikörper, dem Zielmolekül und den zweiten Antikörpern auf dem Träger besteht, wobei der erste Antikörper und der zweite Antikörper an unterschiedliche Epitope des Zielmoleküls binden, einen Nachweisschritt Umsetzen von
zwei oder mehr Substraten mit Spaltungsstellen durch die Enzyme, einer fluoreszierenden Substanz, die an eine endständige Seite von jeder der Spaltungsstellen gebunden ist, und einem Quencher, der an eine andere endständige Seite davon gebunden ist, wobei die fluoreszierenden Substanzen in der Fluoreszenzwellenlänge gegenseitig unterschiedlich sind und
dem Komplex,
um Fluoreszenz, die von den fluoreszierenden Substanzen emittiert wird, nachzuweisen; und
einen Analyseschritt Verarbeiten von zwei oder mehr Nachweissignalen von Fluoreszenz, die sich in der Wellenlänge unterscheidet, als ein Nachweissignal des Zielmoleküls.

2. Nachweisverfahren nach Anspruch 1, wobei der Träger ein Mikrokügelchen ist.

3. Nachweisverfahren nach Ansprüchen 1 oder 2, das ein digitaler ELISA ist.

4. Nachweisverfahren nach Anspruch 3, umfassend, zwischen dem Komplexbildungsschritt und dem Nachweisschritt, einen Umschließungsschritt Umschließen der Träger nacheinander in Tröpfchen, die auf einer Grundplatte gebildet sind.

5. Verfahren zum Nachweis eines Zielmoleküls, umfassend einen Komplexbildungsschritt Umsetzen von
einem Zielmolekül,
einem Partikel, das mit einem ersten Antikörper modifiziert ist, der spezifisch an das Zielmolekül bindet und
zwei oder mehr zweiten Antikörpern, die spezifisch an das Zielmolekül binden und mit gegenseitig unterschiedlichen Markierungen bereitgestellt werden,
um einen Komplex zu bilden, der aus dem ersten Antikörper, dem Zielmolekül und den zweiten Antikörpern auf dem Partikel besteht, wobei der erste Antikörper und der zweite Antikörper an unterschiedliche Epitope des Zielmoleküls binden;
einen Nachweisschritt Nachweisen der Signale von den Markierungen; und
einen Analyseschritt Verarbeiten von zwei oder mehr unterschiedlichen Signalen von den Markierungen als ein Nachweissignal des Zielmoleküls.

6. Nachweisverfahren nach Anspruch 5, das ein digitaler ELISA ist.

7. Nachweisverfahren nach Anspruch 6, umfassend, zwischen dem Komplexbildungsschritt und dem Nachweisschritt, einen Umschließungsschritt Umschließen der Partikel nacheinander in Tröpfchen, die auf einer Grundplatte gebildet sind.

8. Enzymgekoppelter Immunadsorptions-Assay (ELISA) -Kit, umfassend einen Träger, der mit einem ersten Antikörper modifiziert ist, der spezifisch an ein Zielmolekül bindet,
zwei oder mehr zweite Antikörper, die spezifisch an das Zielmolekül binden und mit Enzymen markiert sind, die eine Substratspaltungsaktivität und gegenseitig unterschiedliche Substratspezifitäten aufweisen, wobei der erste Antikörper und der zweite Antikörper an unterschiedliche Epitope des Zielmoleküls binden, und
zwei oder mehr Substrate mit Spaltungsstellen durch die Enzyme, einer fluoreszierenden Substanz, die an eine endständige Seite von jeder der Spaltungsstellen gebunden ist, und einem Quencher, der an eine andere endständige Seite davon gebunden ist, wobei die fluoreszierenden Substanzen in der Fluoreszenzwellenlänge gegenseitig unterschiedlich sind.

## Revendications

1. Procédé pour détecter une molécule cible, comprenant
une étape de formation de complexe consistant à faire réagir
une molécule cible,
un vecteur modifié avec un premier anticorps qui se lie spécifiquement à la molécule cible, et
deux seconds anticorps ou plus qui se lient spécifiquement à la molécule cible et sont marqués avec des enzymes ayant une activité de clivage de substrat et des spécificités de substrat mutuellement différentes
pour former un complexe constitué du premier anticorps, de la molécule cible et des seconds anticorps sur le vecteur, dans lequel le premier anticorps et le second anticorps se lient à des épitopes différents de la molécule cible ;
une étape de détection consistant à faire réagir
deux substrats ou plus ayant des sites de clivage par les enzymes, une substance fluorescente liée à un côté terminal de chacun des sites de clivage et un désactiveur lié à un autre côté terminal de ceux-ci, dans lequel les substances fluorescentes sont mutuellement différentes en terme de longueur d'onde de fluorescence, et
le complexe
pour détecter une fluorescence émise par les substances fluorescentes ; et
une étape d'analyse consistant à traiter deux signaux de détection de fluorescence ou plus différents en terme de longueur d'onde en tant que signal de détection de la molécule cible.

2. Procédé de détection selon la revendication 1, dans lequel le vecteur est une microbille.

3. Procédé de détection selon les revendications 1 ou 2, qui est un procédé ELISA numérique.

4. Procédé de détection selon la revendication 3, comprenant, entre l'étape de formation de complexe et l'étape de détection, une étape d'encloisonnement consistant à encloisonner les vecteurs un par un dans des gouttelettes formées sur une plaque de base.

5. Procédé pour détecter une molécule cible, comprenant
une étape de formation de complexe consistant à faire réagir
une molécule cible,
une particule modifiée avec un premier anticorps qui se lie spécifiquement à la molécule cible, et
deux seconds anticorps ou plus qui se lient spécifiquement à la molécule cible et sont pourvus de marqueurs mutuellement différents
pour former un complexe constitué du premier anticorps, de la molécule cible et des seconds anticorps sur la particule, dans lequel le premier anticorps et le second anticorps se lient à des épitopes différents de la molécule cible ;
une étape de détection consistant à détecter les signaux à partir des marqueurs ; et une étape d'analyse consistant à traiter deux signaux différents ou plus à partir des marqueurs en tant que signal de détection de la molécule cible.

6. Procédé de détection selon la revendication 5, qui est un procédé ELISA numérique.

7. Procédé de détection selon la revendication 6, comprenant, entre l'étape de formation de complexe et l'étape de détection, une étape d'enfermement consistant à enfermer les particules une par une dans des gouttelettes formées sur une plaque de base.

8. Kit de dosage d'immunoabsorption par enzyme liée (ELISA), comprenant
un vecteur modifié avec un premier anticorps qui se lie spécifiquement à une molécule cible,
deux seconds anticorps ou plus qui se lient spécifiquement à la molécule cible et sont marqués avec des enzymes ayant une activité de clivage de substrat et des spécificités de substrat mutuellement différentes, dans lequel le premier anticorps et le second anticorps se lient à des épitopes différents de la molécule cible, et
deux substrats ou plus ayant des sites de clivage par les enzymes, une substance fluorescente liée à un côté terminal de chacun des sites de clivage et un désactiveur lié à un autre côté terminal de ceux-ci, dans lequel les substances fluorescentes sont mutuellement différentes en terme de longueur d'onde de fluorescence.
